# EUROPEAN PATENT SPECIFICATION

(11) **EP 2 166 876 B1**
(45) Date of publication and mention of the grant of the patent: **10.04.2013**
(21) Application number: 08784913.9
(22) Date of filing: 21.07.2008
(51) Int. Cl.: A23L 1/03, A23L 1/304, A61K 38/43, A23L 1/302

(54) **IRON FORTIFICATION NUTRITIONAL BLEND**
ERNÄHRUNGSMISCHUNG FÜR EISENANREICHERUNG
MELANGE NUTRITIONNEL FORTIFIE EN FER

(30) Priority: 25.07.2007 EP 07014577
(43) Date of publication of application: 31.03.2010
(73) Proprietor: DSM IP Assets B.V., 6411 TE Heerlen (NL)
(72) Inventor: KRAEMER, Klaus, 79713 Bad Säckingen (DE); STEIGER, Georg, A-1190 Wien (AT); WIEPRECHT, Torsten, 79650 Schopfheim (DE)
(74) Representative: Schwander, Kuno
(86) International application number: PCT/EP2008/005946
(87) International publication number: WO 2009/012953

(56) References cited:
- EP-A- 0 772 978
- WO-A-02/12882
- WO-A-02/054881
- WO-A-02/098442
- WO-A-03/013283
- WO-A-2004/071218
- CN-A- 101 057 633
- DAVIDSSON ET AL.: "Bioavailability in infants of iron from infant cereals: effect of dephytinization." AMERICAN JOURNAL OF CLINICAL NUTRITION, vol. 65, no. 4, 1997, pages 916-920, XP002503304
- HURRELL R F: "Fortification: overcoming technical and practical barriers" JOURNAL OF NUTRITION, WISTAR INSTITUTE OF ANATOMY AND BIOLOGY, PHILADELPHIA, PA, US, vol. 132, no. 4S, 1 January 2002 (2002-01-01), pages 806S-812S, XP008098049 ISSN: 0022-3166

## Description

The present invention relates to a nutritional blend for the fortification of food products with iron, furthermore it relates to a fortified food product.

Multiple vitamin and/or mineral nutrient (so-called micronutrient) deficiency continues to be highly prevalent in developing countries and is therefore an important public health issue. Iron deficiency (or "sideropenia") is the most common known form of nutritional deficiency. The direct consequence of iron deficiency is iron deficiency anemia. Mainly children, adolescent girls and women, here especially pregnant and breast feeding are affected. Because iron is essential for most plants and animals, a wide range of food can provide it. However, these foods are absorbed and processed differently by the body; for instance, iron from meat (heme iron source) is more easily broken down and absorbed than iron in grains (nonheme iron source), and minerals and chemicals in one type of food may inhibit absorption of iron from another type of food eaten at the same time.

Phytic acid (or phytate when in salt form) is the principal storage form of phosphorus and minerals in many plant tissues, especially bran and seeds. Phytate is found within the hulls of nuts, seeds, and grains. It is a strong chelator of important minerals such as calcium, magnesium, iron and zinc and can therefore contribute to mineral deficiencies in people whose diets rely on these foods for their mineral intake. In this way, phytate is an anti-nutrient.

Phytases are enzymes which split phytic acid (or phytate) in myo-inositol und orthophosphate and therefore increase the bioavailability of occluded minerals. Phytases are - like phytate - found within the hulls of grains and are activated by food processing like soaking, dough processing, fermentation etc.: e.g. about 50 % of the phytate complexes are split during the fermentation process in the making of whole wheat meal bread with yeast. During the production of whole rye meal bread using sourdough even all phytate complexes are split. Besides the food processing techniques themselves also physical parameters like particle size of the flour as well as pH value affect the enzymatic degradation of phytate: finely ground grist, low pH and soaking times of several hours promote the liberalization of minerals.

Unfortunately there are a lot of countries or circumstances where these techniques are not used or known and accordingly more or less unprocessed grain or other high phytate food is consumed. As the diet of refugees or people in other emergencies often contains high phytate levels, they especially suffer from mineral deficiencies. Therefore iron supplementation is often necessary and is accordingly a key for treating malnourished persons.

One way to supply malnourished persons effectively and at relatively low costs with micronutrients are micronutrient rich powder blends, which are added to the food by the end-consumer. Successful trials with various concepts (e.g. Sprinkles^{™} or others) have proven the efficacy of this approach. These formulations supply often very high levels of iron in order to overcome low bio-availability of the used iron form and/or high levels of absorption inhibitors (such as phytate) in the food.

The main disadvantage of these blends is that the iron status of the supplemented person has to be considered to avoid excessive iron intake as excessive iron can be toxic: free ferrous iron reacts with peroxides to produce free radicals, which are highly reactive and can damage DNA, proteins, lipids, and other cellular components. Thus, iron toxicity occurs when there is free iron in the cell, which generally occurs when iron levels exceed the capacity of transferrin to bind the iron.

Especially malaria infected, non-anaemic individuals may be affected by a bolus effect of free iron in the plasma (increased level of non transferrin bound iron in the plasma) if a micronutrient powder with high levels of iron is given together with a diet containing low levels of inhibitors (such as phytate). Iron supplementation has been shown to be safe in iron deficient (not iron replete) individuals living at a high risk area for malaria (Sazawal S., Black R.E., Ramsan M., Chwaya H.M., Stoltzfus R., Dutta A., Dhingra U., Kabole I, Deb S., Othman M.K., Kabole F.M. Effect of routine prophylactic supplementation with iron and folic acid on admission to hospital and mortality in preschool children in a high malaria transmission setting: a community-based, randomized, placebo-controlled trial. Lancet, 2006; 367:133-43). Accordingly, large-scale screening of the iron status under such conditions in developing countries could be a solution to the above mentioned problem, but is unfortunately not feasible for a number of reasons (analytical methods and devices suitable for the field, costs, infection risks, etc).

It was therefore an object of the following invention to provide a micronutrient blend for home fortification that meets the nutritional requirements of all malnourished persons and that can be considered as being safe, independent whether a person has malaria or not, is anaemic or not and/or eats phytate-rich products or not. For all these various conditions a universal, safe, but as well efficient iron concept is required.

WO0212882 discloses a composition containing one or more nutrients (iron), vitamin C and further enzymes (phytase). Moreover, WO0212882 discloses methods of controlling weight by counteracting inhibitory effects of xenobiotics on the weight controlling system of a subject. It does not relate to a method of providing malnourished persons with micronutrients.

WO04071218 discloses a food preparation comprising a phytase, a phytate and an essential cation characterized in that at least part of the essential cation is bound to phytate.

L. Davidsson et al. (Am. J. Clin. Nutr. 1997:65:916-920) disclose a food composition comprising phytase, vitamin C and iron added as FeSO₄.

R.F. Hurrell, (The Journal of Nutrition 2002:132:806-812) discloses the use of iron, vitamin C. phytase, NaFeEDTA individually, but does not give any hints about a specific combination according to the present invention.

EP0772978 discloses feed for animals comprising vitamin C, a phytase and ferro-sulfate heptahydrate.

The WO-02/098442-A2 describes a composition (in particular in the form of a pharmaceutical composition, a medication or a food supplement) comprising at least one phytic acid-splitting compound - preferably a phytase - as well as its use, in particular for the increase of bioavailability of essential bio elements (in particular calcium, magnesium, zinc and/or iron, preferably in the form of their ions), but this publication could still not pave the way to the present invention.

It has surprisingly been found that the object of the present invention is achieved by a nutritional blend suitable for fortifying food containing 0.5 mg to 10 mg iron in form of a constant highly bio-available iron source, 50 FTU to 5000 FTU Phytase and at least 20 mg of Vitamin C, each per one serving; optionally further containing one or more nutrients, characterized in that the bio-available iron source is NaFe³⁺EDTA.

1 FTU (also-called FYT) is the amount of phytase that liberates 1µmole phosphate per minute at pH 5.5 and 37 °C.

It was not to be foreseen by the person skilled in the art that a nutritional blend according to the present invention would solve the above mentioned issues. The invention describes a solution which allows a universal use of the nutritional blend, and hence saves cost and lives concurrently.

According to the present invention the nutritional blend containing the nutrients and the phytase is preferably added as a dry water dispersible powder (so-called "sprinkle") or a liquid preparation to the ready-to-eat phytate containing food (e.g. an already prepared and still warm corn porridge) before consumption, whereby the nutritional blend is mixed thoroughly with the food. This intense mixing is on the one hand beneficial for the efficacy of the phytase and on the other hand minimizes the negative impact on the taste deriving form included nutrients like minerals or specific vitamins. Thus the phytase starts immediately - i.e. already before consumption - to break down phytate and the process continues in the stomach. This procedure increases the efficacy and allows lower phytase levels e.g. compared to the state of the art described in patent WO-02/098442-A2.

Accordingly in one embodiment the invention relates to a method of providing malnourished persons, especially persons in malaria endemic areas, with micronutrients comprising the steps of
a) administering a nutritional blend containing 0.5 mg to 10 mg iron in form of a bio-available iron source and 50 FTU to 5000 FTU Phytase and at least 20 mg Vitamin C, each per daily serving of the blend, optionally further containing one or more nutrients, to a ready-to-eat food immediately before consumption; and
b) thoroughly mixing the blend with the food
wherein, the bio-available iron source is NaFe³⁺EDTA.

The term "immediately before consumption" as used herein denotes a period of time from about 1 minute to about 60 minutes, preferably from about 1 minute to about 30 minutes, more preferred from about 1 minute to about 15 minutes.

The term "phytase" as used herein denotes phosphatases which split phytic acid in *myo*-inositol und orthophosphate. A distinction is drawn between 3-phytase and 6-phytase according to the carbon atom at which the orthophosphate is split off the phytic acid:
3-phytase (Enzyme EC 3.1.3.8):
   *myo*-inositol hexakisphosphate + H₂O = 1 D-*myo*-inositol 1,2,4,5,6-pentakisphosphate + phosphate
6-phytase (Enzyme EC 3.1.3.26):
   *myo*-inositol hexakisphosphate + H₂O = 1 D-*myo*-inositol 1,2,3,4,5-pentakisphosphate + phosphate

According to the present invention 3-phytase food grade, 6-phytase or mixtures thereof are especially preferred. The phytase of the present invention may come from plant and/or microbiological sources. A preferable plant source for phytase is malt.

The nutritional blend of the present invention may be packed either in suitable single serving packages (preferably 1 g) or in bigger units like e.g. 150 g bags (so called "multi serving packages"). In this case calibrated spoons are necessary for proper dosing.

The term "nutrient" as used herein denotes physiologically essential components of the human diet such as vitamins, e.g., vitamin A, vitamin B1, Folic acid, Niacin, vitamin B12, vitamin B2, vitamin B6, vitamin E, C, Biotin, Pantothenates, vitamin K, vitamin D as well as derivatives and mixtures of these, as well as further minerals and trace elements such as selenium, zinc and calcium.

According to the present invention it is advantageous if the amount of nutrients present in the nutritional blend is sufficient to provide about 15 to 300 % of the RDA (Recommended Daily Allowance for an adult) in 1 g or one daily serving of the premix.

The nutrients are usually added in a powdery form, i.e. oily vitamins like vitamin A or vitamin E are preferably used as powdery product forms (e.g. as adsorbates, spray dried powders or bead lets which may contain further ingredients, like matrix components - e.g. hydrocolloids - antioxidants, plasticizers, and/or emulsifiers). Even more preferred are water-dispersible powdery product forms of these nutrients.

The nutritional blend according to the present invention can be used to produce a food composition or a beverage.

The food composition may contain amino acids, other enzymes, protein, flour (e.g. malt flour), PUFA powders and so on. Polyunsaturated fatty acids (PUFAs), which are suitable according to the present invention, are mono- or polyunsaturated carboxylic acids having preferably 16 to 24 carbon atoms and, in particular, 1 to 6 double bonds, preferably having 4 or 5 or 6 double bonds. In a preferred embodiment commercially available ROPUFA® '30' n-3 Food Oil (DSM Nutritional Products Ltd, Kaiseraugst, Switzerland) is used. In another preferred embodiment of the present invention, the PUFA ester is ROPUFA^{®} '75' n-3 EE. ROPUFA '75' n-3 EE is refined marine oil in form of an ethyl ester with minimum content of 72 % n-3 fatty acid ethyl ester. It is stabilized with mixed tocopherols, ascorbyl palmitate, citric acid and contains rosemary extract. In another preferred embodiment of the present invention the PUFA ester is ROPUFA® '10' n-6 Oil, a refined evening primrose oil with minimum 9 % gamma linolenic acid which is stabilized DL-alpha-tocopherol and ascorbyl palmitate.

The beverage may be a base composition to which upon its use water or another liquid beverage composition (such as milk, juice and so on) can or has to be added. The base composition can also be prepared as a concentrate to which water or another liquid beverage composition has to be added, or as a beverage to which no liquid needs to be added.

The amount of nutritional blend according to the present invention which is to be added to a food composition depends on the potency of said nutritional blend, i.e. the amount of iron and optional further nutrients in the nutritional blend.

The invention is further illustrated by the following examples.

### Examples:

Recommended intake:
One serving per person and day mixed into food with high phytate content (e.g. corn, cereals with high extraction rates, soy etc).

### Example 1: Not part of the invention

1 g (= one serving) home fortification blend contains:

| **Nutrient** | **per serving** |
|---|---|
| Vitamin A | 400 µg RE |
| Vitamin D3 | 5 µg |
| Vitamin E | 5 mg TE |
| Vitamin K1 | 30 µg |
| Thiamine | 0.5 mg |
| Riboflavin | 0.5 mg |
| Pyridoxine | 0.5 mg |
| Folate | 150 µg |
| Niacin | 6 mg |
| Vitamin B12 | 0.9 µg |
| Vitamin C | 50 mg |
| Iron | 2 mg |
| Zinc | 4.1 mg |
| Selenium | 17 µg |
| Iodine | 90 µg |
| Phytase | 190 FTU |
| Carriers | |

### Example 2:

1 kg (1000 servings) contains:

| **Nutrient** | **per 1 kg** |
|---|---|
| Vitamin A | 1 733 332 IU |
| Vitamin D | 260 000 IU |
| Tocopherol Equivalent (Vitamin E) | 5 500 mg |
| Vitamin K1 | 39 mg |
| Thiamine (Vitamin B1 Base) | 630 mg |
| Riboflavin (Vitamin B2 | 630 mg |
| Pyridoxine (Vitamin B6 Base) | 630 mg |
| Vitamin C | 60 000 mg |
| Folic Acid Anhydrous | 187 mg |
| Niacinamide | 6 600 mg |
| Vitamin B12 | 1 125 µg |
| Copper | 616 mg |
| Iodine | 108 mg |
| Iron (as NaFe³⁺EDTA) | 2 200 mg |
| Selenium | 18 mg |
| Zinc | 4 510 mg |
| Trisodium Citrate | 10 000 mg |
| Silicon Dioxide | 200 mg |
| Phytase | 380 000 FTU |
| Carrier (Potato Maltodextrin DE 11-14) | ad 1 kg |

### Example 3: Not part of the invention

1 kg (1000 servings) contains:

| **Nutrient** | **per 1 kg** |
|---|---|
| Vitamin A | 1 733 332 IU |
| Vitamin D | 260 000 IU |
| Tocopherol Equivalent (Vitamin E) | 5 500 mg |
| Vitamin K1 | 39 000 µg |
| Thiamine (Vitamin B1 Base) | 630 mg |
| Riboflavin (Vitamin B2 | 630 mg |
| Pyridoxine (Vitamin B6 Base) | 630 mg |
| Vitamin C | 60 000 mg |
| Folic Acid Anhydrous | 187 mg |
| Niacinamide | 6 600 mg |
| Vitamin B12 | 1 125 µg |
| Copper | 616 mg |
| Iodine | 108 mg |
| Iron (as ferric pyrophosphate) | 4 400 mg |
| Selenium | 18 mg |
| Zinc | 4510 mg |
| Trisodium Citrate | 10 000 mg |
| Silicon Dioxide | 200 mg |
| Phytase | 380 000 FTU |
| Carrier (Potato Maltodextrin DE 11-14) | ad 1 kg |

About 1 g of such a blend is added to the food (to one portion) just before consumption:

### Preparation:

100 g corn flour are boiled with 250g water and 1 g salt until the water has been absorbed by the corn. The porridge is cooled down to about 60°C or less. Then the nutritional blend is mixed into the warm porridge. The product can be consumed afterwards.

## Claims

1. Nutritional blend suitable for fortifying food containing 0.5 mg to 10 mg iron in form of a bio-available iron source and 50 FTU to 5000 FTU Phytase and at least 20 mg Vitamin C, each per one serving of the blend; optionally further containing one or more nutrients **characterized in that** the bio-available iron source is NaFe³⁺EDTA.

2. Nutritional blend according to claim 1 **characterized in that** the inclusion level is 1.5 - 3 mg iron per serving and day.

3. Nutritional blend according to any of the preceding claims **characterized in that** the phytase is selected from 3-phytase food grade, 6-phytase or mixtures thereof.

4. Nutritional blend according to any of the preceding claims **characterized in that** it further comprises one or more nutrients selected from vitamin A, vitamin B1, Folic acid, Niacin, vitamin B12, vitamin B2, vitamin B6, vitamin E, Biotin, Pantothenates, vitamin K, vitamin D as well as derivatives and mixtures of these.

5. Non-therapeutic method of providing malnourished persons with micronutrients comprising the steps of
a) administering a nutritional blend containing 0.5 mg to 10 mg iron in form of a bio-available iron source, 50 FTU to 5000 FTU phytase and at least 20 mg Vitamin C, each per one serving of the premix, optionally further containing one or more nutrients, to a ready-to-eat food immediately before consumption; and
b) thoroughly mixing the premix with the food,
wherein, the bio-available iron source is NaFe³⁺EDTA.

6. Food or beverage obtainable according to claim 5.

7. Instant beverage according to claim 6.

8. Nutritional blend as outlined in any of the claims 1 to 4 for use in the prevention and treatment of iron deficiency.

9. Nutritional blend as outlined in any of the claims 1 to 4 for use in supplying humans in malaria endemic areas with the nutritional need of iron (together with the intrinsic iron of the consumed cereals) but without the risk of a bolus effect.

10. Nutritional blend as outlined in any of the claims 1 to 4 for use in supplying infants and children in malaria endemic areas with the nutritional need of iron.

## Patentansprüche

1. Nährstoff-Blend, das sich zum Anreichern von Nahrungsmitteln eignet und das jeweils 0,5 mg bis 10 mg Eisen in Form einer biologisch verfügbaren Eisenquelle und 50 FTU bis 5000 FTU Phytase sowie mindestens 20 mg Vitamin C pro Portion des Blends enthält; und das gegebenenfalls einen oder mehrere Nährstoffe enthält, **dadurch gekennzeichnet, dass** es sich bei der biologisch verfügbaren Eisenquelle um NaFe³⁺EDTA handelt.

2. Nährstoff-Blend nach Anspruch 1, **dadurch gekennzeichnet, dass** die enthaltene Menge 1,5-3 mg Eisen pro Portion pro Tag beträgt.

3. Nährstoff-Blend nach einem der vorhergehenden Ansprüche, **dadurch gekennzeichnet, dass** die Phytase aus der Reihe 3-Phytase in Lebensmittelqualität, 6-Phytase oder Mischungen davon ausgewählt ist.

4. Nährstoff-Blend nach einem der vorhergehenden Ansprüche, **dadurch gekennzeichnet, dass** es weiterhin einen oder mehrere Nährstoffe, ausgewählt aus der Reihe Vitamin A, Vitamin B1, Folsäure, Niacin, Vitamin B12, Vitamin B2, Vitamin B6, Vitamin E, Biotin, Pantothenate, Vitamin K, Vitamin D sowie Derivate und Mischungen davon, umfasst.

5. Nicht therapeutisches Verfahren zum Versorgen von fehlernährten Personen mit Mikronährstoffen, umfassend die folgenden Schritte:
a) Versetzen eines verzehrfertigen Nahrungsmittels unmittelbar vor dem Verzehr mit einem Nährstoff-Blend, das jeweils 0,5 mg bis 10 mg Eisen in Form einer biologisch verfügbaren Eisenquelle und 50 FTU bis 5000 FTU Phytase sowie mindestens 20 mg Vitamin C pro Portion des Blends enthält; und das gegebenenfalls einen oder mehrere Nährstoffe enthält; und
b) gründliches Vermischen der Vormischung mit dem Nahrungsmittel,
wobei es sich bei der biologisch verfügbaren Eisenquelle um NaFe³⁺EDTA handelt.

6. Nahrungsmittel oder Getränk, das nach Anspruch 5 herstellbar ist.

7. Instant-Getränk nach Anspruch 6.

8. Nährstoff-Blend wie in einem der Ansprüche 1 bis 4 umrissen, für die Verwendung bei der Vorbeugung und Behandlung von Eisenmangel.

9. Nährstoff-Blend wie in einem der Ansprüche 1 bis 4 umrissen, für die Verwendung beim Versorgen von Menschen in Gebieten, in denen Malaria endemisch ist, mit ihrem ernährungsphysiologischen Bedarf an Eisen (zusammen mit dem in den verzehrten Getreidewaren vorhandenen Eisen) ohne die Gefahr eines Bolus-Effekts.

10. Nährstoff-Blend wie in einem der Ansprüche 1 bis 4 umrissen, für die Verwendung zum Versorgen von Kindern, einschließlich Kleinkindern, in Gebieten, in denen Malaria endemisch ist, mit ihrem ernährungsphysiologischen Bedarf an Eisen.

## Revendications

1. Mélange nutritionnel convenable pour la fortification d'aliments, contenant de 0,5 mg à 10 mg de fer sous forme d'une source de fer biodisponible, de 50 FTU à 5000 FTU de phytase et au moins 20 mg de Vitamine C, chacun pour une portion du mélange ; contenant éventuellement en outre un ou plusieurs nutriments, **caractérisé en ce que** la source de fer biodisponible est le NaFe³⁺EDTA.

2. Mélange nutritionnel selon la revendication 1, **caractérisé en ce que** le degré d'incorporation est de 1,5-3 mg de fer par portion et par jour.

3. Mélange nutritionnel selon l'une quelconque des revendications précédentes, **caractérisé en ce que** la phytase est choisie parmi la 3-phytase de qualité alimentaire, la 6-phytase, ou des mélanges de celles-ci .

4. Mélange nutritionnel selon l'une quelconque des revendications précédentes, **caractérisé en ce qu'**il comprend en outre un ou plusieurs nutriments choisis parmi la vitamine A, la vitamine B1, l'acide folique, la niacine, la vitamine B12, la vitamine B2, la vitamine B6, la vitamine E, la biotine, les pantothénates, la vitamine K, la vitamine D, ainsi que des dérivés et des mélanges de ceux-ci.

5. Méthode non thérapeutique d'apports de micronutriments à des personnes sous-alimentées, comprenant les étapes consistant à :
a) administrer un mélange nutritionnel contenant de 0,5 mg à 10 mg de fer sous forme d'une source de fer biodisponible, de 50 FTU à 5000 FTU de phytase et au moins 20 mg de Vitamine C, chacun pour une portion du prémélange ; contenant éventuellement en outre un ou plusieurs nutriments, à un aliment prêt à consommer juste avant consommation ; et
b) mélanger soigneusement le prémélange avec l'aliment ;
**caractérisé en ce que** la source de fer biodisponible est le NaFe³⁺EDTA.

6. Aliment ou boisson pouvant être obtenu(e) selon la revendication 5.

7. Boisson instantanée selon la revendication 6.

8. Mélange nutritionnel tel qu'exposé selon l'une quelconque des revendications 1 à 4, pour une utilisation dans la prévention et le traitement de la déficience en fer.

9. Mélange nutritionnel tel qu'exposé selon l'une quelconque des revendications 1 à 4, pour une utilisation dans l'apport, à des êtres humains dans des zones endémiques de paludisme, du besoin nutritionnel en fer (conjointement avec le fer intrinsèque des céréales consommées), mais sans risque d'effet bolus.

10. Mélange nutritionnel tel qu'exposé selon l'une quelconque des revendications 1 à 4, pour une utilisation dans l'apport, à des nourrissons et des enfants dans des zones endémiques de paludisme, du besoin nutritionnel en fer.
